# EUROPEAN PATENT APPLICATION

(11) **EP 4 644 534 A1**
(43) Date of publication of application: **05.11.2025**
(21) Application number: 23910307.0
(22) Date of filing: 20.12.2023
(51) Int. Cl.: C12N 5/071

(54) **SCALE EXTRACTION METHOD FOR EXOSOMES IN FRESH MILK**

(30) Priority: 29.12.2022 CN 202211711663
(71) Applicant: Shaanxi Huikang Bio-Tech Co., Ltd., Xi'an, Shaanxi 710065 (CN)
(72) Inventor: WANG, Xin, Xi'an, Shaanxi 710065 (CN); WANG, Ruili, Xi'an, Shaanxi 710065 (CN); ZHAI, Jianghua, Xi'an, Shaanxi 710065 (CN); DENG, Han, Xi'an, Shaanxi 710065 (CN); CHENG, Nanqiong, Xi'an, Shaanxi 710065 (CN); LUO, Linna, Xi'an, Shaanxi 710065 (CN); LI, Yuan, Xi'an, Shaanxi 710065 (CN)
(74) Representative: Habermann, Hruschka & Schnabel
(86) International application number: PCT/CN2023/140378
(87) International publication number: WO 2024/140378

(57) **Abstract**

A method for large-scale extraction of exosomes from fresh milk including: filtering fresh milk by using a hydrophilic and lipophobic nano-interfacial net membrane to remove cell fragments and fat particles in the fresh milk, and performing ultrafiltration by means of a tangential flow ultrafiltration device using an ultrafiltration membrane having a pore size of 100 kDa to 300 kDa to concentrate the clarified fresh milk; mixing the concentrated liquid with an aqueous two-phase solution prepared by combining polyethylene glycol and dextran, then performing phase separation, collecting an enriched exosome solution in the lower phase, and repeating one or two times to concentrate exosomes; further separating the concentrated exosomes by means of a column using a highly cross-linked agarose filler to remove other impurity proteins, and separating out exosomes and external vesicles having different diameters. The method includes simple purification process, without the need for high-speed centrifugation, and tens liters or more of fresh milk can be processed at a time, the extraction purity is high, external vesicles having various particle sizes can be effectively separated, and a large-scale extraction can be achieved with minimal damage to exosomes.

## Description

### CROSS-REFERENCE TO RELATED APPLICATIONS

The present application claims priority of Chinese Patent Application No. 202211711663.5, filed on December 29, 2022 and entitled "METHOD FOR LARGE-SCALE EXTRACTION OF EXOSOMES FROM FRESH MILK", the contents of which are incorporated herein by reference in its entity.

### TECHNICAL FIELD

The present invention pertains to the technical field of extraction processes, and specifically relates to a method for large-scale extraction of exosomes from fresh milk.

### BACKGROUND ART

Exosomes are a type of metabolite with a lipid bilayer membrane released by cells, which appear round or cup-shaped under transmission electron microscopy, with a diameter of 30-150 nm. A large number of studies have shown that exosomes contain various components such as proteins, nucleic acids, and lipids. It is found in current studies that exosomes have very promising application prospects, as they contain abundant markers and can be applied to disease monitoring, treatment and other aspects. Moreover, exosomes derived from different tissues and cells all possess the characteristics of their source tissues and cells, and play a key role in the process of exosome-mediated intercellular material and information exchange. At the same time, exosomes have broad application prospects in clinical treatment, carrier drugs, clinical detection and other aspects. Components such as proteins, lipids, RNA, and miRNA in tumor cell exosomes may be used for the diagnosis and prediction of cancer.

A large number of exosomes have also been found in the currently studied fresh milk. Existing studies have shown that fresh milk exosomes contribute to immune regulation, to regulate intestinal development, and to alleviate some diseases. The domestic research on fresh milk exosomes mainly focuses on the extraction and identification of exosomes, as well as studies on their use as drug carriers and disease screening markers. whereas foreign studies on cow milk exosomes are more extensive, which also involve human immune system, intestinal development, cancer prevention, obesity, and risks brought by the development of chronic diseases such as diabetes. Recently, the research on fresh milk exosomes has become more and more in-depth. As a product, the research orientation and the demand for functional studies are also increasing growing. Therefore, method for extracting large quantities of exosomes from fresh milk needs to be solved.

At present, there are various existing technologies for extracting exosomes, mainly including ultracentrifugation, kit method, and sucrose density gradient centrifugation is the most commonly used means for exosome extraction and purification. The quantity extracted by these methods is limited, the process is also time-consuming, the requirements for equipment are high, the recovery rate is limited, and the purity does not meet the requirements. The above methods cannot solve the requirements for large-large-scale extraction and purification, which limits the application range of exosome industrialization.

There are also few separation and extraction methods of exosomes from fresh milk reported in literature. The existing various extraction methods also have advantages and disadvantages, especially in terms of large-scale extraction, which is too limited to achieve mass production. Moreover, the milk fat in fresh milk is dispersed in the milk plasma in a state of fat globules, generally with a diameter of 0.1-10 µm, and the vast majority in the range of 2-5 µm. The biggest obstacle affecting the extraction of exosomes is the fat particles, which is generally comprised in an amount of 3%-5% in fresh milk, and it is insoluble in water, dispersed in fresh milk in the form of microspheres to form an emulsion, thus cannot be removed by ordinary filtration. Therefore, it is of particular importance to identify a method for large-scale extraction of exosomes from fresh milk with low cost, high efficiency and wide applicability, which can simultaneously remove fat particles.

### SUMMARY OF THE INVENTION

The object of the present invention is to provide a method for large-scale extraction of exosomes from fresh milk in the view of the problems in the technology of fresh milk exosome extraction.

To achieve the abovementioned object, the present invention provides a method for large-scale extraction of exosomes from fresh milk, including: filtering the fresh milk by a hydrophilic and lipophobic nano-interfacial net membrane to remove cell fragments and fat particles from the fresh milk, and then performing ultrafiltration by means of a tangential flow ultrafiltration device with an ultrafiltration membrane having a pore size of 100-300 kDa to concentrate the clarified fresh milk; performing phase separation after mixing the concentrated liquid with an aqueous two-phase solution prepared by combining polyethylene glycol and dextran, collecting an enriched exosome solution in the lower phase, and repeating 1-2 times to concentrate the exosomes; further separating the concentrated exosomes by a column using highly cross-linked agarose filler to remove other impurity proteins, and separating out the exosomes and external vesicles having various diameters. The method includes simple purification process, without the need for high-speed centrifugation, and tens liters or more of fresh milk can be processed at a time, the extraction purity is high, external vesicles having various particle sizes can be effectively separated, and a large-scale extraction can be achieved with minimal damage to exosomes.

Specifically, the present invention provides a method for large-scale extraction of exosomes from fresh milk comprising the following steps:
1. The fresh milk is filtered by a hydrophilic and lipophobic nano-interfacial net membrane to remove fat particles from the fresh milk, and filtrate is collected.
2. The filtrate collected in step 1 was subjected to an ultrafiltration by means of a tangential flow ultrafiltration device with an ultrafiltration membrane having a pore size of 100-300 kDa to concentrate the filtrate from which fat particles have been removed, and concentrated fresh milk is obtained.
3. The concentrated fresh milk collected in step 2 is thoroughly mixed with an aqueous two-phase solution and subjected to centrifugal separation, the upper phase is discarded, and the lower phase is collected. Such step is repeated for 1-2 times for the lower phase to further concentrate a solution of the exosome. The aqueous two-phase solution is prepared by dissolving polyethylene glycol and dextran in distilled water or PBS buffer, with the concentration of polyethylene glycol in the aqueous two-phase solution being 20-40 g/L and the concentration of dextran being 14-20 g/L.
4. After the concentrated exosome solution in step 3 is diluted with a diluting solution, it is further separated through a gel column, which utilizes highly cross-linked agarose as the filler, and the diluting solution is a 20-50 mM Tris-HCl buffer with pH=7.2 containing 0-150 mM NaCl. Specifically, in some embodiments, the diluting solution is a 20 mM Tris-HCl buffer with pH=7.2 containing 150 mM NaCl.

In the above step 1, the fresh milk includes any one or more of fresh cow milk, fresh goat or sheep milk, fresh human milk, and camel milk.

In the above step 1, the hydrophilic and lipophobic nano-interfacial net membrane has a coating, which is a polyvinyl alcohol/silica nano-composite membrane.

In the above step 1, the membrane has a contact angle with water of 0°-25°, and a contact angle with fat in fresh milk of 60°-130°, which can be used to prevent fat particles in fresh milk from passing through the membrane.

In the above step 2, the concentrated fresh milk has a concentration factor of 10-20 times.

In the above step 2, the ultrafiltration membrane is a polyethersulfone ultrafiltration membrane.

In the above step 3, it is preferable to thoroughly mix the concentrated fresh milk collected in step 2 with the aqueous two-phase solution in a volume ratio of 1:3-3:1, and then perform centrifugal separation.

In the above step 3, the polyethylene glycol has a molecular weight of 5 kDa-35 kDa, and the dextran has a molecular weight of 7 kDa-65 kDa.

In the above step 3, preferably, the polyethylene glycol has a molecular weight of 25 kDa-35 kDa, and the dextran has a molecular weight of 45 kDa-50 kDa.

In the above step 3, the centrifugal force for the centrifugal separation is 1000-3000 g, and the centrifugation duration is 10-40 minutes.

In the above step 4, the highly cross-linked agarose has a molecular weight of 600 kDa-800 kDa and a particle size of 75-95 µm.

The present invention has following beneficial effects:
1. The present invention first filters fresh milk by a hydrophilic and lipophobic nano interface net membrane to collect a clarified solution and to remove fat particles in fresh milk, which solves the problem of the impact of fat on purification. Then, ultrafiltration is performed using a tangential flow ultrafiltration device with an ultrafiltration membrane having a pore size of 100 kDa ~300 kDa for the first concentration, to remove some small-molecule proteins and water, so that the clarified fresh milk is concentrated by 10-20 times. The clarified filtrate of fresh milk after concentration is subjected to centrifugal separation with an aqueous two-phase solution prepared by combining polyethylene glycol and dextran for the second concentration and preliminary purification, whereby exosomes are compressed into the dextran phase, and the upper phase is discarded. After repeating this process 1-2 times, the clarified exosomes are concentrated by 60-150 times, converting production-level quantity into laboratory-level quantity. The exosomes are then highly concentrated, making a gel column separation possible. Finally, a gel column with highly cross-linked agarose as the filler is used for separation to remove other impurity proteins and apoptotic bodies, and exosomes and extracellular vesicles with different particle sizes are purified, to realize large-scale extraction of exosomes from fresh milk.
2. The entire operation process of the present invention is simple, avoids the limitation of high-speed centrifugation on extraction scale, thus is suitable for large-scale extraction and concentration, overcome the problem of limited sample loading amount of the gel column through nearly 100-fold high concentration, and solves, and solve the difficulty of large-scale extraction. No chemical reagents with strong denaturing ability or violent reactions are introduced in the entire experimental process, causing minimal damage to exosomes. No ultracentrifuge or immunomagnetic beads are required, thus industrial production can be achieved without complicated process. A large amount of exosomes from various sources can be processed. The extracted exosomes may undergo a series of identifications and verifications such as activity identification, electron microscopic assay, particle size analysis, nanoparticle tracking analysis, and protein content detection, with low damage rate, low mutation rate, and stable properties.

### BRIEF DESCRIPTION OF THE DRAWINGS

Fig. 1 is an image showing a microscopic observation of fresh cow milk before removing fat particles (a), after removing fat particles using a hydrophilic and lipophobic nano-interfacial net membrane (b), and after removing fat particles using a chemical reagent precipitation method (c).
Fig. 2 is a particle size distribution diagram obtained by particle size analysis (NTA) verification after ultrafiltration concentration by the tangential flow ultrafiltration device in Example 1.
Fig. 3 is a particle size distribution image obtained by particle size analysis (NTA) verification of exosomes extracted by the aqueous two-phase solution in Example 1.
Fig. 4 is a transmission electron microscopy (TEM) image verifying exosomes extracted by the gel column in Example 1.
Fig. 5 is a particle size distribution image obtained by particle size analysis (NTA) verification of exosomes extracted by the gel column in Example 1.
Fig. 6 is a distribution diagram of vesicles with various sizes after separation by the highly cross-linked agarose gel column in Example 1.
Fig. 7 is a diagram (western blot) verifying the substances of Peak 1 and Peak 2 in Fig. 6 using an exosome protein marker CD63.

### DETAILED DESCRIPTION OF EMBODIMENTS

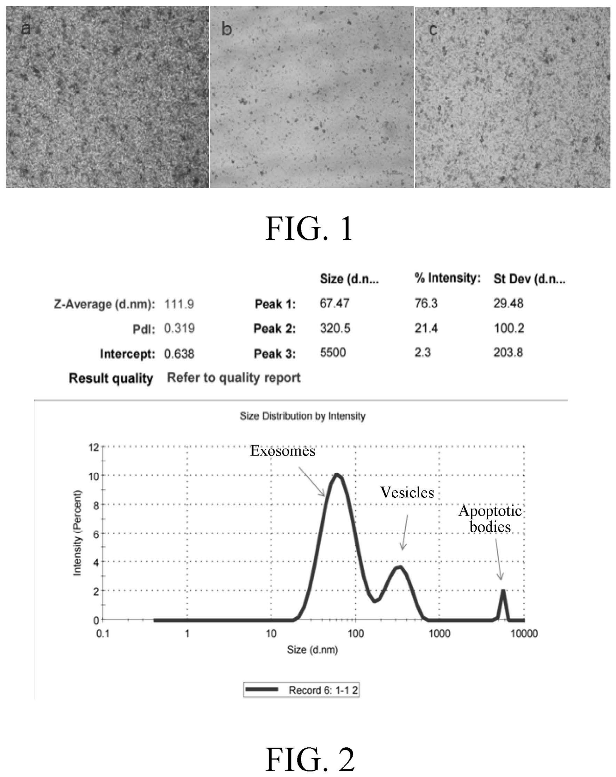

First, taking fresh cow milk as an example, in the present invention, the effects of a hydrophilic and lipophobic nano-interfacial net membrane (coated with a polyvinyl alcohol/silica composite membrane) in separating and removing fat particles was compared with those of chemical reagent precipitation. The experiment was specifically performed as follows: 1000 mL of fresh cow milk was filtered through a hydrophilic and lipophobic nano-interfacial net membrane coated with a polyvinyl alcohol/silica composite membrane to remove fat particles in the fresh cow milk, and the permeate was collected. Another 1000 mL of fresh cow milk was placed in a beaker, heated to 40°C, added with 0.2 M pH 4.6 sodium acetate buffer solution while shaking, adjusted to pH 4.8 using NaOH aqueous solution with a mass concentration of 1% or acetic acid aqueous solution with a volume concentration of 10%, cooled to room temperature, left standing for 5 minutes, filtered through filter paper to remove precipitates, and then filtered through a microfiltration (MF) membrane having a pore size of 1.4 µm to remove most of fat particles, and the permeate was collected. Comparing images a, b, and c in Fig. 1 with each other, it can be seen that the polyvinyl alcohol/silica composite membrane removes fat particles more thoroughly and has a better effect.

Next, taking fresh cow milk as an example, in the present invention, the influence of ordinary ultrafiltration and tangential flow ultrafiltration methods on the concentration effect of exosomes was investigated. The experiment was specifically performed as follows: 1000 mL of fresh cow milk was filtered through a hydrophilic and lipophobic nano-interfacial net membrane coated with a polyvinyl alcohol/silica composite membrane to remove fat particles in the fresh cow milk, and the filtrate was collected. A part of the collected filtrate was ultrafiltered through an ordinary ultrafiltration membrane having a pore size of 0.45 µm (with a pressure lower than that of 0.22 µm), the ultrafiltrate was collected, and then filtered through an ultrafiltration membrane having a pore size of 100 kDa to remove some small-molecule proteins and water, and the concentrated liquid on the ultrafiltration membrane was collected. The liquid was concentrated by 15 times, but the pressure increased during the filtration, making it difficult to perform subsequent filtration, the filter membrane was blocked by the solution, and the filter needed to be replaced midway. Another part of the collected filtrate was ultrafiltered through a tangential flow ultrafiltration device with polyethersulfone ultrafiltration membrane having a pore size of 100 kDa to remove some small-molecule proteins and water, and the concentrated liquid on the ultrafiltration membrane was collected. The liquid was concentrated by nearly 10 times, and the concentration duration was shortened by more than half compared with that of the ordinary ultrafiltration. The concentrated liquid was further mixed with an aqueous two-phase solution (formulated by fully dissolving polyethylene glycol with a molecular weight of 35 kDa and dextran with a molecular weight of 50 kDa in distilled water, in which the concentration of polyethylene glycol was 33.5 g/L and the concentration of dextran was 16.5 g/L) in a volume ratio of 1:1, centrifuged at 3000 g for 20 minutes, the upper phase was discarded, the lower phase solution was collected, and the collected lower phase was mixed with the aqueous two-phase solution in a volume ratio of 1:1, and the centrifugal separation was repeated once to remove about 90% of the liquid in the upper phase. Finally, the remaining liquid was concentrated by about 125 times relative to fresh cow milk. The exosome solution concentrated by the aqueous two-phase system had basically met the experimental requirements, as demonstrated by electron microscope identification and particle size analysis.

Then, taking fresh cow milk as an example, in the present invention, the influence of changes in the pore size of the ultrafiltration membrane during the ultrafiltration process of the tangential flow ultrafiltration device on the concentration effect of exosomes was investigated. The experiment was specifically performed as follows: for each group, 2000 mL of fresh cow milk was filtered through a hydrophilic and lipophobic nano-interfacial net membrane coated with a polyvinyl alcohol/silica composite membrane to remove fat particles in the fresh cow milk, the filtrate was collected, and then the collected filtrate was ultrafiltered through a tangential flow ultrafiltration device with polyethersulfone ultrafiltration membranes of various pore sizes, as shown in Table 1, to remove a part of small-molecule proteins and water. The effects of ultrafiltration were shown in Table 1.

**Table 1: Comparison of ultrafiltration results using ultrafiltration membranes with different pore sizes**

| Ultrafiltration pore size | 300 kDa | 100 kDa | 10 kDa |
|---|---|---|---|
| Concentration factor | 13 times concentrated | 10 times concentrated | 8 times concentrated |
| Filtration time | 30 min | 45 min | 90 min |
| Average Particle Size of Exosomes | 130±3.0 nm | 134±10.5 nm | 143±18.2 nm |
| Number of Exosomes | 3.07×10¹³ | 3.30×10¹³ | 5.2×10¹³ |
| Concentration results | Fast speed, high efficiency | Slower concentration speed, significantly reduced efficiency | Longer concentration time, increased impurity particles, slightly increased average particle size |

It can be seen from the experimental results in Table 1 above that the pore size of 300 kDa selected for the ultrafiltration membrane resulted in the best concentration effect. As the pore size of the ultrafiltration membrane decreased, the ultrafiltration duration was prolonged and the efficiency became poor, but it exhibited little effect on the extraction results and was able to meet the experimental requirements. When the ultrafiltration pore size of 10 kDa was selected, in addition to the abovementioned characteristics, the particle size of the extracted exosomes was increased, and an increase in impurity particles was detected. Therefore, an ultrafiltration membrane having a pore size of 100 kDa to 300 kDa was selected in the present invention.

Finally, taking fresh cow milk as an example, in the present invention, the influence of aqueous two-phase solutions composed of polyethylene glycol (PEG) and dextran (DEX) having different molecular weights on the concentration effect of exosomes was investigated. The experiment was specifically performed as follows: for each group, 200 mL of fresh cow milk was filtered through a hydrophilic and lipophobic nano-interfacial net membrane coated with a polyvinyl alcohol/silica composite membrane to remove fat particles in the fresh cow milk, the filtrate was collected, and then the collected filtrate was ultrafiltered through a tangential flow ultrafiltration device with a polyethersulfone ultrafiltration membrane having a pore size of 300 kDa to remove some small-molecule proteins and water, and the concentrated liquid on the ultrafiltration membrane was collected. The concentrated liquid was mixed in a volume ratio of 1:1 with an aqueous two-phase solution (prepared by fully dissolving PEG and DEX in distilled water, in which the concentration of PEG was 33.5 g/L and the concentration of DEX was 16.5 g/L) composed of PEG and DEX having different molecular weights as shown in Table 2, centrifuged at 3000 g for 20 minutes, the upper phase was discarded, and the exosome solution recovered in the lower phase dextran was collected. The recovery rate of exosomes was shown in Table 2 below.

**Table 2: Recovery rate of exosomes extracted by aqueous two-phase solutions composed of PEG and DEX with different molecular weights**

| Recovery Rate % | PEG 5 kDa | PEG 8 kDa | PEG 10 kDa | PEG 20 kDa | PEG 25 kDa | PEG 30 kDa | PEG 35 kDa |
|---|---|---|---|---|---|---|---|
| DEX 7 kDa | 50% | 52% | 54% | 55% | 64% | 60% | 55% |
| DEX 10 kDa | 53% | 53% | 56% | 57% | 66% | 67% | 58% |
| DEX 45 kDa | 55% | 60% | 60% | 65% | 64% | 70% | 70% |
| DEX 50 kDa | 53% | 55% | 58% | 62% | 64% | 68% | 75% |
| DEX 65 kDa | 52% | 55% | 58% | 60% | 57% | 59% | 59% |

Note: The recovery rate in the table refers to the volume concentration of exosomes in the lower phase.

It can be seen from Table 2 above that the aqueous two-phase solution prepared by the combination of PEG 35 kDa and DEX 50 kDa has the highest recovery rate for extracting exosomes, and the recovery rates of the rest of the combinations gradually decrease with the change of molecular weight, but all meet the use requirements.

The present invention will be further described in detail below in conjunction with the accompanying drawings and Examples, but the scope of the present invention is not limited thereto.

### Example 1

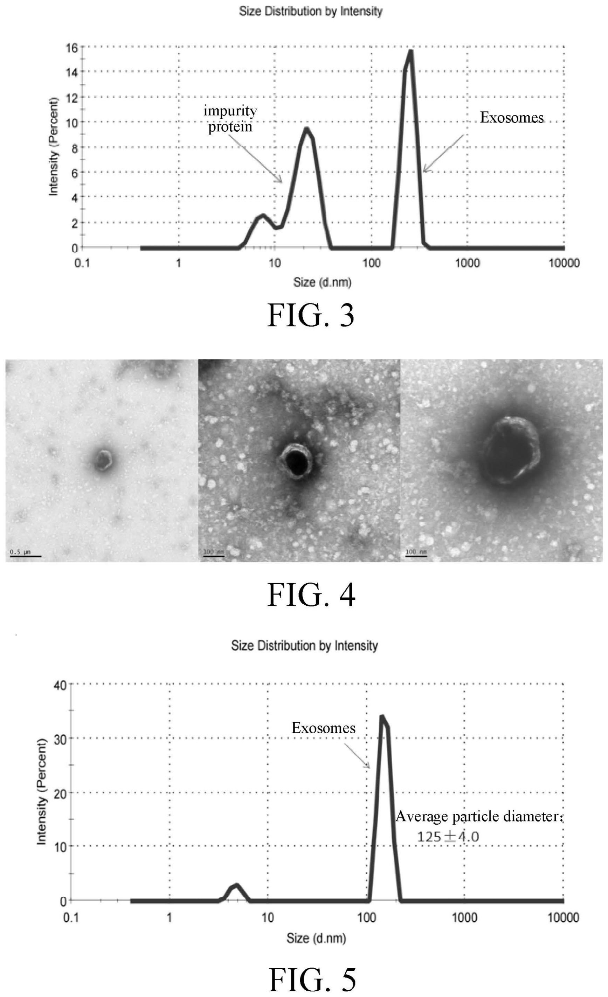

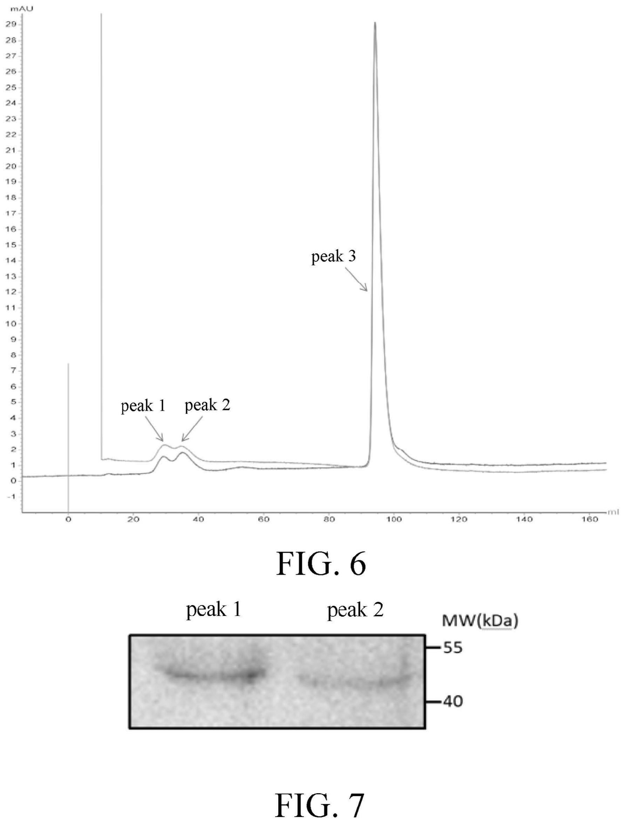

Taking fresh cow milk as an example, exosomes from fresh cow milk were extracted.
1. 2000 mL of fresh cow milk was filtered through a hydrophilic and lipophobic nano-interfacial net membrane coated with a polyvinyl alcohol/silica composite membrane to remove fat particles in the fresh cow milk, and the filtrate was collected.
2. The filtrate collected in step 1 was subjected to ultrafiltration by means of a tangential flow ultrafiltration device with a polyethersulfone ultrafiltration membrane having a pore size of 100 kDa to remove a part of small-molecule proteins and water, and about 200 mL of ultrafiltration concentrate was collected, so that the liquid was concentrated by nearly 10 times. As can be seen from Fig. 2, there were three peaks in the extracted exosomes after ultrafiltration concentration, which were verified to be exosomes, vesicles, and apoptotic bodies.
3. 13.4 g of polyethylene glycol with a molecular weight of 35 kDa and 6.6 g of dextran with a molecular weight of 50 kDa were fully dissolved in distilled water and fixed to 400 mL to prepare an aqueous two-phase solution, in which the concentration of polyethylene glycol was 33.5 g/L and the concentration of dextran was 16.5 g/L. Then, the ultrafiltration concentrate collected in step 2 was mixed with the aqueous two-phase solution in a volume ratio of 1:1, centrifuged at 3000 g for 20 minutes, the upper phase was discarded, and about 40 mL of the lower phase solution was collected. The collected lower phase was then mixed with the aqueous two-phase solution in a volume ratio of 1:1, and the centrifugal separation was repeated once to remove about 80% of the liquid in the upper phase. Finally, 13 mL of liquid remained, which was concentrated by about 150 times relative to fresh cow milk. From the particle size analysis in Fig. 3, after extraction by the aqueous two-phase solution, the former peak represented impurity protein, and the latter peak represented exosomes.
4. The chromatograph was connected to a gel column packed with highly cross-linked agarose having a molecular weight of 600-800 kDa and a particle size of 75-95 µm. Before sample loading, the column was equilibrated with phosphate buffer (0.1 mol/L, pH 7.2) at a flow rate of 0.5 mL/min, and after the baseline of the conductivity and ultraviolet absorption value was stable, the 13 mL of exosome solution remaining after concentration in step 3 was diluted to 30 mL with 20 mM Tris-HCl buffer (pH=7.2) containing 150 mM NaCl and then loaded, with sample loading flow rate being 0.5 mL/min until a permeate peak occurred. Each separation peak was collected, where the first and second peaks were both target peak exosomes, and the third peak was extracellular vesicles. After collection, the column was cleaned with a column washing solution (isopropanol aqueous solution with a volume concentration of 30% containing 1 M NaOH). The exosome solution from each collected peak was identified, and the rest was aliquoted and stored at -80°C. As can be seen from the electron microscope detection in Fig. 4 and the particle size analysis in Fig. 5, the extracted exosomes had an average particle size of 125+4.0 nm and were high in purity. The extracted exosome solution was further passed through a gel column using cross-linked agarose as the filler to obtain Fig. 6. The analysis indicated that both peak 1 and peak 2 separated were exosomes, and peak 3 was vesicles secreted by cells. The exosomes extracted from peak 1 and peak 2 were verified by the protein marker CD63. As can be seen from the western blot in Fig. 7, both peak 1 and peak 2 were exosomes.

### Example 2

In step 3 of Example 1, the ultrafiltration concentrate collected in step 2 was mixed with the aqueous two-phase solution in a volume ratio of 3:1, centrifuged at 3000 g for 20 minutes, the upper phase was discarded, and because the lower phase was in a large proportion, about 200 mL of the lower phase solution was collected. The collected lower phase was further mixed with the aqueous two-phase solution in a volume ratio of 3:1, and the centrifugal separation was repeated twice to remove the liquid in the upper phase. Finally, 33 mL of liquid remained, which was concentrated by about 60 times relative to fresh cow milk. Other steps were the same as in Example 1, the identification results were in conformity with the characteristics of exosomes, the electron microscope detection was the same as that in Fig. 4, and the average particle size was 129±5 nm.

### Example 3

In step 3 of Example 1, the ultrafiltration concentrate collected in step 2 was mixed with the aqueous two-phase solution in a volume ratio of 1:3, centrifuged at 3000 g for 20 minutes, the upper phase was discarded, and the lower phase solution was collected to about 100 mL. The collected lower phase was further mixed with the aqueous two-phase solution in a volume ratio of 1:3, and the centrifugal separation was repeated twice to remove the liquid in the upper phase. Finally, 20 mL of liquid remained, which was concentrated by about 100 times relative to fresh cow milk. Other steps were the same as in Example 1, the identification results were in conformity with the characteristics of exosomes, the electron microscope detection was the same as that in Fig. 4, and the average particle size was 130±5 nm.

## Claims

1. A method for large-scale extraction of exosomes from fresh milk, wherein the method comprises the following steps:
(1) filtering the fresh milk through a hydrophilic and lipophobic nano-interfacial net membrane to remove fat particles from the fresh milk, and collecting the filtrate;
(2) ultrafiltering the filtrate collected in step (1) using a tangential flow ultrafiltration device with a 100 to 300 kDa pore-size ultrafiltration membrane to concentrate the filtrate from which fat particles have been removed, thereby obtaining concentrated fresh milk;
(3) thoroughly mixing the concentrated fresh milk collected in step (2) with an aqueous two-phase solution, centrifuging the mixture for separation, discarding the upper phase, and collecting the lower phase; and repeating this procedure 1 to 2 times for the lower phase to further concentrate a solution of the exosome; wherein the aqueous two-phase solution is prepared by dissolving polyethylene glycol and dextran in distilled water or a PBS buffer, with polyethylene glycol having a concentration of 20 to 40 g/L and dextran having a concentration of 14 to 20 g/L;
(4) diluting the concentrated solution of exosomes obtained from step (3) with a diluting solution, and then further separating it through a gel column using highly cross-linked agarose as the a filler, wherein the diluting solution is a 20 mM Tris-HCl buffer with pH=7.2 containing 150 mM NaCl.

2. The method for large-scale extraction exosomes from fresh milk according to claim 1, wherein in step (1), the fresh milk includes any one or more of fresh cow milk, fresh goat or sheep milk, fresh human milk, and camel milk.

3. The method for large-scale extraction of exosomes from fresh milk according to claim 1, wherein in step (1), the hydrophilic and lipophobic nano-interfacial net membrane has a coating, with the coating being a polyvinyl alcohol/silica nano-composite membrane.

4. The method for large-scale extraction of exosomes from fresh milk according to claim 3, wherein in step (1), the polyvinyl alcohol/silica nano-composite membrane has a contact angle with water of 0°-25°, and a contact angle with fat in fresh milk of 60°-130°.

5. The method for large-scale extraction of exosomes from fresh milk according to claim 1, wherein in step (2), the concentrated fresh milk has a concentration factor of 10-20 times.

6. The method for large-scale extraction of exosomes from fresh milk according to claim 1, wherein in step (2), the ultrafiltration membrane is a polyethersulfone ultrafiltration membrane.

7. The method for large-scale extraction of exosomes from fresh milk according to claim 1, wherein in step (3), the concentrated fresh milk collected in step (2) is thoroughly mixed with the aqueous two-phase solution in a volume ratio of 1:3-3:1, and then centrifuged for separation.

8. The method for large-scale extraction of exosomes from fresh milk according to claim 1, wherein in step (3), the polyethylene glycol has a molecular weight of 5 kDa-35 kDa, and the dextran has a molecular weight of 7 kDa-65 kDa.

9. The method for large-scale extraction of exosomes from fresh milk according to claim 1 or 4, wherein in step (3), the centrifuging is performed at a centrifugal force of 1000-3000 g for 10-40 minutes.

10. The method for large-scale extraction of exosomes from fresh milk according to claim 1, wherein in step (4), the highly cross-linked agarose has a molecular weight of 600 kDa-800 kDa and a particle size of 75-95 µm.
